# EUROPEAN PATENT SPECIFICATION

(11) **EP 2 881 174 B1**
(45) Date of publication and mention of the grant of the patent: **28.06.2017**
(21) Application number: 13195426.5
(22) Date of filing: 03.12.2013
(51) Int. Cl.: B01J 37/03, B01J 29/14, C07C 2/84

(54) **Catalyst composition for the production of styrene**
Katalysatorzusammensetzung zur Herstellung von Styrol
Composition de catalyseur pour la production de styrène

(43) Date of publication of application: 10.06.2015
(73) Proprietor: Saudi Basic Industries Corporation, Riyadh 11422 (SA)
(72) Inventor: Das, Debasish, 391775 Gujarat (IN); Attarwala, Onen Amiruddin, 56225 Bangalore (IN)
(74) Representative: Modiano, Micaela Nadia

(56) References cited:
- WO-A1-2013/106040
- US-A- 4 483 936
- US-A1- 2013 237 735

## Description

The present invention is directed to a process for the production of a catalyst composition, such catalyst composition and the use of such catalyst composition for the production of styrene.

Current major commercial process practiced globally for styrene production is through catalytic dehydrogenation of ethylbenzene. This is a very mature technology and a large part of the world's styrene capacity is produced through this route. The starting material ethylbenzene is produced through alkylation reaction between high purity benzene and ethylene and they make up a very large part of styrene's cost of production.

There are a few developing routes for styrene production, of which alkylation of toluene with methanol in presence of a basic catalyst seems to be attractive as both toluene and methanol are much cheaper than the usual feedstock benzene and ethylene. However, in this alternative route selectivity towards styrene was reported to be not so high and ethylbenzene was reported to be the major product.

The alkylation of the side chain of toluene with methanol or formaldehyde by contact of these reactants with X- or Y-type zeolites is described in Yashima et al in the Journal of Catalysis, Vol. 26, 303-312 (1972). More specifically, it is disclosed therein that alkylation of the methyl group of toluene to form styrene and ethylbenzene is effected by Na, K, Rb or Cs exchanged X- or Y-type zeolites, whereas Li exchanged zeolites of the same type effected predominantly alkylation of the benzene ring of toluene to form xylenes. Yashima et al interpret their results as suggesting that xylene formation is attributable to the acidity of the catalyst, whereas styrene and ethylbenzene formation is attributable to the basicity of the catalyst. The problem with this process is the low selectivity towards styrene.

JP57-68144 is directed to catalyst for styrene synthesis which comprises a zeolite of the faujasite class having at least 20% of the sodium cations present therein exchanged with cesium, potassium or rubidium and which has been treated to impregnate therein one or more divalent or trivalent metal salts of boric or phosphoric acid, the metal of said salt disclosed as being selected from magnesium, calcium, aluminum, manganese, iron, cobalt, nickel, copper and zinc.

US4483936 describes a catalyst composition capable of catalyzing the side chain alkylation of toluene with methanol comprising a crystalline aluminosilicate zeolite of the faujasite structure having present therein (1) at least one alkali metal selected from the group consisting of Cs, K, and Rb; (2) at least one metal selected from the Group of M metals consisting of Mn, Fe, Cu, and Zn; and (3) at least one member selected from the group consisting of boron and phosphorus.

US 2013/237735 A1 discloses a method for preparing a catalyst by preparing zirconium borate and mixing it with Cs13X zeolite, wherein the zirconium borate was prepared by adding a zirconium nitrate solution to a solution of disodium tetraborate under continuous stirring.

WO 2013/106040 A1 discloses a method for preparing a catalyst comprising a zeolite comprising a first promotor Cs and a second promotor and third promotor, wherein the third promotor may be Co or B.

It is an objective of the present invention to provide a catalyst composition for toluene production with an improved selectivity.

Accordingly, the present invention provides a process for the preparation of a catalyst composition comprising the steps of:
A) preparing a salt of boric acid or phosphoric acid and a bivalent or trivalent metal and
B) mixing the salt obtained by step A) with a crystalline aluminosilicate zeolite of the faujasite structure with SiO₂/Al₂O₃ mole ratio in the range of about 2 to about 8 and including potassium, rubidium or cesium cations or combinations thereof,
wherein step A) comprises the sub-steps of
A1) mixing a borate or a phosphate of an alkali metal and a water soluble salt of the bivalent or trivalent metal to obtain a suspension,
A2) adding an alkaline solution to the suspension obtained by sub-step A1) to obtain a solution having a pH of 8.5-11.5 and
A3) mixing the solution obtained by sub-step A2) to obtain the salt.

It was found that the production process of styrene from toluene using the catalyst composition obtained according to the process of the invention shows a surprisingly high selectivity towards styrene.

### Sub-step A)

In step A), a salt of boric acid or phosphoric acid and a bivalent or trivalent metal is prepared.

The phosphoric acid and boric acid which forms the salt may be of any suitable form. As the phosphoric acid, orthophosphoric acid, pyrophosphoric acid, metaphosphoric acid, etc. may be used, orthophosphoric acid being most preferred. As the boric acid, orthoboric acid, metaboric etc. may be used, orthoboric acid being most preferred. The salt of step A) is preferably a borate of the bivalent or trivalent metal.

The bivalent or trivalent metal which forms the salt is preferably selected from the group consisting of magnesium, calcium, aluminum, chromium, manganese, iron, cobalt, nickel, copper, zinc, lanthanum, cerium and zirconium. Most preferably, the bivalent or trivalent metal is cobalt.

The following are preferred examples of the salt: magnesium borate, calcium borate, aluminum borate, chromium borate, manganese borate, iron borate, cobalt borate, nickel borate, copper borate, zinc borate, magnesium phosphate, calcium phosphate, aluminum phosphate, chromium phosphate, manganese phosphate, iron phosphate, cobalt phosphate, nickel phosphate, copper phosphate and zinc phosphate. These metal salts may also be used by mixing two or more types. Preferably, the metal salt is or comprises cobalt borate.

### Sub-step A1)

In sub-step A1), a borate or a phosphate of an alkali metal and a water soluble salt of the bivalent or trivalent metal are mixed to obtain a suspension. Preferably, the borate or the phosphate of the alkali metal of sub-step A1) comprises disodium tetraborate. The water soluble salt of the bivalent or trivalent metal may be any suitable salt, but preferably is an acetate.

### Sub-step A2)

The pH of the suspension obtained by sub-step A1) is adjusted to 8.5-11.5 by the addition of an alkaline solution. It was found that this range of pH resulted in a catalyst composition with a high selectivity towards styrene. More preferably, the pH is adjusted to 8.5-10.5, which results in an even higher selectivity towards styrene. Even more preferably, the pH is adjusted to 8.5-9.5 or 8.8-9.2.

The alkaline solution may be of any type, including alkali hydroxides such as sodium hydroxide.

### Sub-step A3)

The solution obtained by sub-step A2) is mixed until the salt is obtained, for example by stirring the solution for 5-6 hours. The obtained salt may be filtered, washed and dried.

### Step B)

The salt obtained by step A) is mixed with a crystalline aluminosilicate zeolite of the faujasite structure with SiO₂/Al₂O₃ mole ratio in the range of about 2 to about 8 and including potassium, rubidium or cesium cations or combinations thereof.

### Zeolite

The zeolite used in the present invention preferably has a molar ratio of SiO₂/Al₂O₃ within the range of approximately 2:1 and approximately 8:1, exemplified by synthesized faujasite referred to as type X and type Y. An example of the type X and type Y faujasite is, respectively:
Type X: Na₃₆ {(AlO₂)₈₆ (SiO₂)₁₀₆}·276H₂O
Type Y: Na₃₆ {(AlO₂)₈₆ (SiO₂)₁₂₉}·276H₂O.

The zeolite is preferably type X, in particular 13X zeolite.

The method for replacing some or most sodium ions in zeolite with potassium, rubidium or cesium cations is well known, as described e.g. in US4115424. The following is given as one example:
Cesium hydroxide (CsOH) aqueous solution (125 g CsOH dissolved in 1650 ml water) is added to 75 g type X zeolite (for example, molecular sieve Na13X) in several batches and ion exchange is carried out for 10 to 48 hours while heating at 80 to 90°C. Subsequently, the zeolite treated with ion-exchange is washed well with water and dried for a day in a dryer at 130 to 150°C. The zeolite obtained in this manner generally has 10 to 50% of the sodium ions thereof replaced by cesium ions.

Preferably, the ion exchanged zeolite includes cesium cations.

### Mixing metal salt and ion-exchanged zeolite

The weight ratio between the metal salt obtained in step A) and the zeolite which are mixed in step B) is typically between 1:100 and 30:100, preferably between 5:100 and 25:100.

There are no particular restrictions with regards to the method for mixing the metal salt and the ion-exchanged zeolite. For example, the ion-exchanged zeolite may be mixed with a solution of the metal salt or a suspension of the metal salt. Preferably, the metal salt and the ion-exchanged zeolite are mixed in a dry state e.g. in a mortar. Subsequently, the mixture may be calcined at e.g. 400 to 550°C for e.g. 2 to 4 hours prior to using this for a styrene synthesis reaction.

### Preferred embodiments

In particularly preferred embodiments, step A) comprises the sub-steps of
A1) mixing disodium tetraborate and cobalt acetate to obtain a suspension,
A2) adding a solution of sodium hydroxide to the suspension obtained by sub-step A1) to obtain a solution having a pH of 8.5 - 11.5 and
A3) mixing the solution obtained by sub-step A2) to obtain the salt.

### Catalyst composition

The present invention further provides the catalyst composition obtainable by the process according to the invention.

### Styrene production

The present invention further provides a process for the production of styrene comprising reacting toluene and an agent selected from methanol and formaldehyde and mixtures thereof at an elevated reaction temperature in the presence of the catalyst composition according to the invention. Preferably, the agent is methanol.

The production of styrene is carried out by a gaseous mixture of toluene and methanol and/or formaldehyde passing through the catalyst zone under thermal conditions. The reaction is preferably conducted at gaseous hourly space velocity of 10 - 1500 hr⁻¹, preferably 15-100 hr⁻¹.

The reaction may be carried out at a molar ratio between toluene and methanol and/or formaldehyde of 1:1, but is preferably carried out at an excess of toluene over methanol and/or formaldehyde. The molar ratio between toluene and the agent selected from methanol and formaldehyde and mixtures thereof may preferably be 1:1 to 20:1, preferably 2:1 to 10:1.

The temperature at which the gaseous mixture contacts the catalyst is preferably within a range of 350 to 500°C, more preferably between 400 to 475°C. Other reaction conditions may be determined according to known methods.

### Use

According to a further aspect, the present invention provides use of the catalyst composition according to the invention for the production of styrene, preferably from toluene and methanol and/or formaldehyde.

Although the invention has been described in detail for purposes of illustration, it is understood that such detail is solely for that purpose and variations can be made therein by those skilled in the art without departing from the spirit and scope of the invention as defined in the claims.

It is further noted that the invention relates to all possible combinations of features described herein, preferred in particular are those combinations of features that are present in the claims.

It is further noted that the term 'comprising' does not exclude the presence of other elements. However, it is also to be understood that a description on a product comprising certain components also discloses a product consisting of these components. Similarly, it is also to be understood that a description on a process comprising certain steps also discloses a process consisting of these steps.

The invention is now elucidated by way of the following examples, without however being limited thereto.

### Examples

### Example 1

### Preparation of Cs-13X

Cesium exchanged 13X zeolite (Cs-13X) was prepared by multiple exchange of parent zeolite with cesium hydroxide solution. About 20 g of 13X zeolite in sodium form was suspended in a 300 mL solution (75 g cesium hydroxide/Liter water) and stirred at 100°C for 2 hrs. The solid was filtered, dried and re-suspended again in 300 mL solution of cesium hydroxide and stirred for another 3 hrs. The process was repeated again with the remaining 400 mL of the solution and stirring continued overnight. Finally, the solid was filtered, washed and dried at 110°C.

### Preparation of Cobalt borate

Cobalt borate was prepared as follows: To a 500 mL 0.3M solution of di-sodium tetraborate a solution of cobalt acetate (0.3M, 500 mL) was added drop-wise at room temperature with constant stirring to get a pink suspension. To this a solution of sodium hydroxide (15 g in 40 mL water) was added drop-wise till pH of the resultant solution reached 9. The mixture was stirred for 5-6 hours and the solid was filtered, washed and dried at 120°C for overnight.

### Preparation of Cs-13X + Cobalt borate

The final catalyst was prepared by grinding of 10 g Cs-13X zeolite and 2 g of Cobalt borate in dry state in a mortar. The mixture was calcined at 500°C for 3 hours.

### Reaction of toluene and methanol

The reaction of toluene and methanol was carried out in a down flow fixed bed reactor. Typically 2 g of final catalyst was well mixed with alpha-alumina (1:1 w/w) and loaded in the reactor. The catalyst was pretreated for 2 hours at 375°C in flowing nitrogen after which reactants (toluene and methanol 5:1 molar ratio) were introduced with a liquid feed pump. Typical liquid feed rate was 9.6 mL/h. Nitrogen flow was about 50 mL/min. The reaction temperature was varied from 375-425°C. The feed and reaction products were analyzed by on-line gas chromatograph. The result is given in Table 1.

### Example 2

Example 1 was repeated but cobalt borate was prepared at pH of 11 instead of 9. The result is given in Table 1.

### Comparative experiment A

Example 1 was repeated but Cs-13X was used as the catalyst in the reaction of toluene and methanol. The result is given in Table 1.

### Comparative experiment B

Example 1 was repeated but Cesium and boron exchanged 13X zeolite (Cs-B-13X) was used as the catalyst in the reaction of toluene and methanol. The result is given in Table 1.

Cs-B-13X was prepared by multiple exchange of parent zeolite with a solution of cesium hydroxide and boric acid. About 20 g of 13X zeolite in sodium form was suspended in a 300 mL solution (75 g cesium hydroxide + 55 g boric acid/Liter water) and stirred at 100°C for 2 hrs. The solid was filtered, dried and re-suspended again in 300 mL solution of cesium hydroxide and stirred for another 3 hrs. The process was repeated again with the remaining 400 mL of the solution and stirring continued overnight. Finally, the solid was filtered, washed, dried at 110°C and then calcined at 500°C for 3 hours.

**Table 1: Catalytic performance comparison after 6 hrs at 375°C**

| Catalyst | CEx A Cs-13X | CEx B Cs-B-13X | Ex 1 Cs-13X + Co Borate (pH= 9) | Ex 2 Cs-13X + Co Borate (pH=11) |
|---|---|---|---|---|
| Methanol Conversion (%) | 31.6 | 34.5 | 36.4 | 57.3 |
| Selectivity (mol%) | | | | |
| C1-C2 | 0 | 29 | 0 | 0 |
| Benzene | 0 | 0 | 0 | 2.5 |
| Ethylbenzene | 86.4 | 27.7 | 14.6 | 37 |
| Styrene | 12.0 | 41.4 | 85.4 | 54.2 |
| Xylenes | 0 | 0 | 0 | 0 |
| C₉₊ | 1.6 | 1.9 | 0 | 6.3 |
| Styrene yield | 3.8 | 14.3 | 31.1 | 31.1 |
| Ethylbenzene yield | 27.3 | 9.5 | 5.3 | 21.2 |

In presence of all catalysts listed in Table 1 toluene reacts with methanol at 375°C to give styrene and ethylbenzene. However, selectivity to styrene was different. While Cs-13X showed only 12% styrene selectivity (CEx A), this was improved to 41% with the incorporation of boron but about 30% C1-C2 light fractions were also produced (CEx B). On the other hand, incorporation of Co-borate improved styrene selectivity significantly to 54.2% (Ex 2) or 85% (Ex 1). No other by-products like benzene or xylenes were detected.

It can be seen from Table 1 that cobalt borate samples prepared at a pH 9 showed higher styrene selectivity than samples prepared at pH 11. Further, the ethylbenzene selectivity is also low at 14.6% at pH 9 compared to 37.0% at pH 11. This shows that incorporation of cobalt borate to Cs-13X improved styrene selectivity and styrene yield in a significant way with minimal formation of ethylbenzene by the use of appropriate pH during the preparation of cobalt borate. Formation of larger quantities of ethylbenzene requires further dehydrogenation at around 600-650°C to obtain styrene. Thus, a catalyst which results in higher amounts of styrene with minimum ethylbenzene formation is most desirable.

Excess toluene and unreacted methanol can be recycled back to the reactor. The catalyst showed little deactivation and was found to be quite stable till 10-12 hours of time on stream.

## Claims

1. A process for the preparation of a catalyst composition comprising the steps of:
A) preparing a salt of boric acid or phosphoric acid and a bivalent or trivalent metal and
B) mixing the salt obtained by step A) with a crystalline aluminosilicate zeolite of the faujasite structure with SiO₂ /Al₂O₃ mole ratio in the range of 2 to 8 and including potassium, rubidium or cesium cations or combinations thereof,
wherein step A) comprises the sub-steps of
A1) mixing a borate or a phosphate of an alkali metal and a water soluble salt of the bivalent or trivalent metal to obtain a suspension,
A2) adding an alkaline solution to the suspension obtained by sub-step A1) to obtain a solution having a pH of 8.5-11.5 and
A3) mixing the solution obtained by sub-step A2) to obtain the salt.

2. The process according to claim 1, wherein the salt of step A) comprises the borate of the bivalent or trivalent metal.

3. The process according to any one of the preceding claims, wherein the bivalent or trivalent metal is selected from the group consisting of magnesium, calcium, aluminum, chromium, manganese, iron, cobalt, nickel, copper, zinc, lanthanum, cerium and zirconium.

4. The process according to any one of the preceding claims, wherein the bivalent or trivalent metal comprises cobalt.

5. The process according to any one of the preceding claims, wherein the borate or the phosphate of the alkali metal of step A1) comprises disodium tetraborate.

6. The process according to any one of the preceding claims, wherein the alkaline solution of step A2) is a solution of sodium hydroxide.

7. The process according to any one of the preceding claims, wherein the zeolite is type X, preferably 13X zeolite.

8. The process according to any one of the preceding claims, wherein the zeolite includes cesium cations.

9. The process according to any one of the preceding claims, wherein the pH of step A2) is 8.5-9.5, more preferably 8.8-9.2.

10. The process according to any one of the preceding claims, wherein step A) comprises the sub-steps of
A1) mixing disodium tetraborate and cobalt acetate to obtain a suspension,
A2) adding a solution of sodium hydroxide to the suspension obtained by sub-step A1) to obtain a solution having a pH of 8.5 - 11.5 and
A3) mixing the solution obtained by sub-step A2) to obtain the salt.

11. The catalyst composition obtainable by the process according to any one of the preceding claims.

12. A process for the production of styrene comprising reacting toluene and an agent selected from methanol and formaldehyde and mixtures thereof at an elevated reaction temperature in the presence of the catalyst composition according to claim 11.

13. The process according to claim 12, wherein the reaction is conducted at temperatures in the range of 350°C to 500°C.

14. The process according to claim 12 or 13, wherein the reaction is conducted at gaseous hourly space velocity of 10 - 1500 hr⁻¹, preferably 15-100 hr⁻¹ and an excess of toluene over methanol or formaldehyde.

## Patentansprüche

1. Ein Verfahren für die Herstellung einer Katalysatorzusammensetzung, das die folgenden Schritte umfasst:
A) Herstellen eines Salzes von Borsäure oder Phosphorsäure und eines bivalenten oder trivalenten Metalls und
B) Mischen des durch Schritt A) erhaltenen Salzes mit einem kristallinen Aluminosilikatzeolith der Faujasitstruktur mit einem SiO₂/Al₂O₃ Molverhältnis in dem Bereich von 2 bis 8 und einschließlich Kalium, Rubidium oder Cäsium -Kationen oder Kombinationen daraus,
worin Schritt A) die folgenden Unterschritte umfasst:
A1) Mischen eines Borats oder eines Phosphats eines Alkalimetalls und eines wasserlöslichen Salzes des bivalenten oder trivalenten Metalls, um eine Suspension zu erhalten,
A2) Hinzufügen einer alkalischen Lösung zu der durch Unterschritt A1) erhaltenen Suspension, um eine Lösung mit einem pH von 8,5-11,5 zu erhalten, und
A3) Mischen der durch Unterschritt A2) erhaltenen Lösung, um das Salz zu erhalten.

2. Das Verfahren gemäß Anspruch 1, worin das Salz von Schritt A) das Borat des bivalenten oder trivalenten Metalls umfasst.

3. Das Verfahren gemäß irgendeinem der vorhergehenden Ansprüche, worin das bivalente oder trivalente Metall gewählt ist aus der Gruppe bestehend aus Magnesium, Kalzium, Aluminium, Chrom, Mangan, Eisen, Cobalt, Nickel, Kupfer, Zink, Lanthan, Cer und Zirconium.

4. Das Verfahren gemäß irgendeinem der vorhergehenden Ansprüche, worin das bivalente oder trivalente Metall Cobalt umfasst.

5. Das Verfahren gemäß irgendeinem der vorhergehenden Ansprüche, worin das Borat oder das Phosphat des Alkalimetalls von Schritt A1) Dinatriumtetraborat umfasst.

6. Das Verfahren gemäß irgendeinem der vorhergehenden Ansprüche, worin die alkalische Lösung von Schritt A2) eine Lösung von Natriumhydroxid ist.

7. Das Verfahren gemäß irgendeinem der vorhergehenden Ansprüche, worin der Zeolith Typ X ist, vorzugsweise 13X Zeolith.

8. Das Verfahren gemäß irgendeinem der vorhergehenden Ansprüche, worin der Zeolith Cäsium-Kationen einschließt.

9. Das Verfahren gemäß irgendeinem der vorhergehenden Ansprüche, worin der pH von Schritt A2) 8,5-9,5 ist, bevorzugter 8,8-9,2.

10. Das Verfahren gemäß irgendeinem der vorhergehenden Ansprüche, worin Schritt A die folgenden Unterschritte umfasst:
A1) Mischen von Dinatriumtetraborat und Cobaltacetat, um eine Suspension zu erhalten,
A2) Hinzufügen einer Lösung von Natriumhydroxid zu der durch Unterschritt A1) erhaltenen Suspension, um eine Lösung mit einem pH von 8,5-11,5 zu erhalten, und
A3) Mischen der durch Unterschritt A2) erhaltenen Lösung, um das Salz zu erhalten.

11. Die Katalysatorzusammensetzung, erhältlich durch das Verfahren gemäß irgendeinem der vorhergehenden Ansprüche.

12. Ein Verfahren für die Produktion von Styren umfassend das Reagieren von Toluen und einem Wirkstoff gewählt aus Methanol und Formaldehyd und Mischungen daraus, bei einer erhöhten Reaktionstemperatur in der Anwesenheit der Katalysatorzusammensetzung gemäß Anspruch 11.

13. Das Verfahren gemäß Anspruch 12, worin die Reaktion bei Temperaturen in dem Bereich von 350°C bis 500°C durchgeführt wird.

14. Das Verfahren gemäß Anspruch 12 oder 13, worin die Reaktion bei einer stündlichen Gasraumgeschwindigkeit von 10-1500 Std.⁻¹, vorzugsweise 15-100 Std.⁻¹, und einem Überschuss an Toluen über Methanol oder Formaldehyd durchgeführt wird.

## Revendications

1. Procédé de préparation d'une composition de catalyseur comprenant les étapes suivantes :
A) la préparation d'un sel d'acide borique ou d'acide phosphorique et d'un métal bivalent ou trivalent et
B) le mélange du sel obtenu à l'étape A) avec une zéolite d'aluminosilicate cristallin de structure faujasite présentant un rapport molaire SiO₂/Al₂O₃ dans la plage de 2 à 8 et incluant des cations de potassium, de rubidium ou de césium ou des combinaisons de ceux-ci,
dans lequel l'étape A) comprend les sous-étapes de
A1) mélange d'un borate ou d'un phosphate d'un métal alcalin et d'un sel soluble dans l'eau du métal bivalent ou trivalent pour obtenir une suspension,
A2) ajout d'une solution alcaline à la suspension obtenue par la sous-étape A1) pour obtenir une solution ayant un pH de 8,5 à 11,5 et
A3) mélange de la solution obtenue par la sous-étape A2) pour obtenir le sel.

2. Procédé selon la revendication 1, dans lequel le sel de l'étape A) comprend le borate du métal bivalent ou trivalent.

3. Procédé selon l'une quelconque des revendications précédentes, dans lequel le métal bivalent ou trivalent est choisi dans le groupe constitué du magnésium, du calcium, de l'aluminium, du chrome, du manganèse, du fer, du cobalt, du nickel, du cuivre, du zinc, du lanthane, du cérium et du zirconium.

4. Procédé selon l'une quelconque des revendications précédentes, dans lequel le métal bivalent ou trivalent comprend le cobalt.

5. Procédé selon l'une quelconque des revendications précédentes, dans lequel le borate ou le phosphate du métal alcalin de l'étape A1) comprend le tétraborate de disodium.

6. Procédé selon l'une quelconque des revendications précédentes, dans lequel la solution alcaline de l'étape A2) est une solution d'hydroxyde de sodium.

7. Procédé selon l'une quelconque des revendications précédentes, dans lequel la zéolite est une zéolite de type X, de préférence une zéolite 13X.

8. Procédé selon l'une quelconque des revendications précédentes, dans lequel la zéolite inclut des cations de césium.

9. Procédé selon l'une quelconque des revendications précédentes, dans lequel le pH de l'étape A2) est de 8,5 à 9,5, plus préférablement de 8,8 à 9,2.

10. Procédé selon l'une quelconque des revendications précédentes, dans lequel l'étape A) comprend les sous-étapes de
A1) mélange de tétraborate de disodium et d'acétate de cobalt pour obtenir une suspension,
A2) ajout d'une solution d'hydroxyde de sodium à la suspension obtenue par la sous-étape A1) pour obtenir une solution ayant un pH de 8,5 à 11,5 et
A3) mélange de la solution obtenue par la sous-étape A2) pour obtenir le sel.

11. Composition de catalyseur pouvant être obtenue par le procédé selon l'une quelconque des revendications précédentes.

12. Procédé de production de styrène comprenant la réaction de toluène et d'un agent choisi parmi le méthanol et le formaldéhyde et les mélanges de ceux-ci à une température de réaction élevée en présence de la composition de catalyseur selon la revendication 11.

13. Procédé selon la revendication 12, dans lequel la réaction est réalisée à des températures dans la plage de 350 °C à 500 °C.

14. Procédé selon la revendication 12 ou 13, dans lequel la réaction est réalisée à une vitesse spatiale horaire gazeuse de 10 à 1 500 h⁻¹, de préférence de 15 à 100 h⁻¹ et avec un excès de toluène par rapport au méthanol ou au formaldéhyde.
